# FASCICULE DE BREVET EUROPEEN

(11) **EP 2 881 920 B1**
(45) Date de publication et mention de la délivrance du brevet: **30.11.2016**
(21) Numéro de dépôt: 13290306.3
(22) Date de dépôt: 09.12.2013
(51) Int. Cl.: G08B 17/10, G08B 29/16, G01N 33/00

(54) **Ensemble de détection de fumée comprenant des détecteurs en redondance**
Rauchmeldeanlage, die Sensoren in Redundanzschaltung umfasst
Smoke-detection assembly including redundant detectors

(43) Date de publication de la demande: 10.06.2015
(73) Titulaire: Siemens Schweiz AG, 8047 Zürich (CH)
(72) Inventeur: Frere, Loic, 78000 Versailles (FR)
(74) Mandataire: Maier, Daniel Oliver

(56) Documents cités:
- US-A1- 2004 056 765
- US-A1- 2007 103 325

## Description

La présente invention concerne principalement un ensemble de détection de fumée comprenant des détecteurs en redondance selon le préambule de la revendication 1.

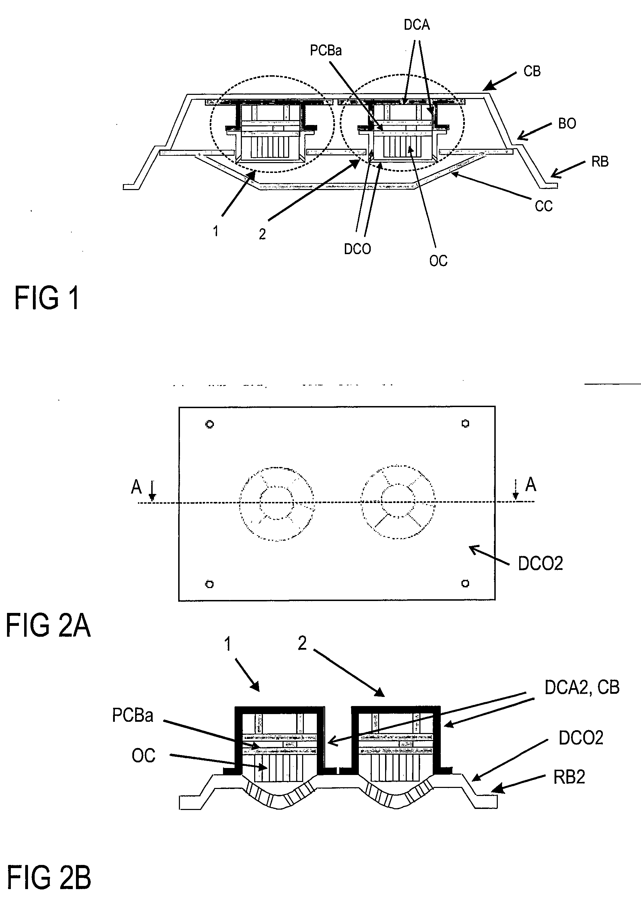

Un ensemble connu de détection de fumée comprenant des détecteurs en redondance est représenté à la figure 1. Actuellement, de tels ensembles sont disposés dans des locaux divers ainsi que dans des véhicules, tels que des avions, en particulier en cabine et surtout en soute afin d'assurer un haut niveau de sécurité de détection de fumée, si un des détecteurs devait être défectueux. Principalement, de tels ensembles sont installés comme un élément plafonnier venant ainsi se loger dans des structures aménagées tels que des faux plafonds ou autres structures de soutien de paroi.

De tels ensemble ont aussi pour buts de détecter des types divers de « fumée » afin de protéger contre des agressions extérieures spécifiques d'un lieu spécifique tels que voué à un feu, un brouillard salin, un insecticide, etc. En effet, les détecteurs de fumée actuels permettent des détections dites multi-critères et le terme « fumée » doit être compris comme un gaz ou un mélange gaz-liquide affectant l'atmosphère localement. Au même titre, d'autres types de détection sont aussi possibles tels que par des moyens de détection/mesure de température ou de pression selon les cas.

Plus précisément selon l'exemple de la figure 1, un tel ensemble de détection de fumée comprend :
- deux détecteurs de fumée (1, 2) identiques et juxtaposés horizontalement, chacun des détecteurs étant composé au moins d'un capteur de fumée couplé et disposé sous une chambre de mesure (OC) reliée à un module électronique (PCBa), la dite chambre étant munie de parois (DCA) latérales ainsi que supérieure formant ainsi un carter de protection de ladite chambre et du module,
- les dits détecteurs sont enfin encapsulés ou assemblés dans une cavité rigide ayant une base supérieure (CB) et un couvercle inférieur (CC), le dit couvercle étant disposé face aux capteurs de fumée et possédant ici deux jeux d'ouvertures (DCO) permettant aux capteurs de détecter une introduction de fumée dans la cavité.

Le couvercle (CC) est fixé sous un bord périphérique interne (BO) à la base (CB) et la dite base présente un rebord externe (RB) au dit bord (BO), le dit rebord permettant de venir fixer l'ensemble complet de détection sur une structure de maintien tel que sous forme de plafonnier.

A partir d'un ensemble tel que celui de la figure 1, un but de la présente invention est de proposer un ensemble de détection de fumée comprenant des détecteurs en redondance pour lequel le dimensionnement et le poids de l'ensemble sont réduits.

Egalement, il est important de pouvoir associer à cet ensemble une méthode de fixation aisée dans une structure portante telle que dans un plafond de bâtiment ou une paroi interne de véhicule/train/avion/etc.

Un tel ensemble est proposé au travers de la revendication 1 ainsi qu'une méthode de fixation de l'ensemble au travers de la revendication 11.

Un ensemble de sous-revendications présente également des avantages de l'invention.

Un exemple de réalisation de l'ensemble de détection selon l'invention est fourni à l'aide des figures suivantes.
- Figures 2A, 2B : vue de dessous et coupe de face d'un premier ensemble de détection selon l'invention,
- Figures 3A, 3B : vue de dessous et coupe de face d'un second ensemble de détection selon l'invention.

Figures 2A et 2B présentent respectivement une vue de dessous et coupe A-A de face d'un premier ensemble de détection selon l'invention comprenant un couvercle (DCO2) monobloc.

A partir d'un ensemble de détection de fumée comprenant :
- deux détecteurs de fumée (1, 2) identiques et juxtaposés horizontalement, chacun composé au moins d'un capteur de fumée disposé sous et couplé à une chambre de mesure (OC) munie de parois (DCA, DCA2) latérales et supérieure,
- les dits détecteurs étant encapsulés dans une cavité rigide ayant une base supérieure (CB) et un couvercle inférieur (CC, DCO, DCO2) disposé face aux capteurs de fumée,
   l'invention prévoit que la base (CB) est exclusivement formée par les parois (DCA2) de chacune des deux chambres de mesure.

De la sorte, une base (CB) de forte dimension telle que présentée à la figure 1 peut être évitée ou du moins est considérablement réduite en taille et en poids, puisqu'elle est remplacée avantageusement par les parois déjà existantes des chambres de mesure. Ainsi aussi, la taille globale (initialement donnée par les dimensions de la base (CB) de forte dimension telle que présentée à la figure 1) de l'ensemble selon l'invention est ainsi réduite, de même pour son poids.

A titre d'exemples, la cavité a pour dimension maximale hors tout de 480*480*60mm et le poids maximal de l'ensemble est de l'ordre de 1.5kg.

La base (CB) sous forme des parois (DCA2) comporte aussi un matériau résistant aux agressions extérieures spécifiques du lieu (feu, brouillard salins, insecticide..) où l'ensemble est présent, en particulier ce matériau peut être en fibre de verre ou de carbone, ce qui permet de diminuer considérablement le poids de la dite base/parois. Il peut en être de même pour le couvercle (DCO2) qui comporte un matériau résistant aux agressions extérieures spécifiques du lieu (feu, brouillard salins, insecticide..) où l'ensemble est présent, particulier en fibre de verre ou de carbone.

Les détecteurs de fumée comprennent généralement des capteurs optiques de faibles masse et pouvant être combinés avec d'autres types de capteurs tels que de type gaz, thermique, pression, etc., selon les besoins de la détection souhaitée.

Il est à noter qu'avantageusement, le couvercle (DCO2) possède un pourtour (RB2) plus étendu qu'un pourtour de la base (DCA2). C'est au moyen de ce pourtour qu'il est possible de fixer l'ensemble de détection complet à un emplacement de maintien tel que sous forme de plafonnier. Toutefois, sachant que les figures 1 et 2A, 2B sont sensiblement à la même échelle, on peut constater facilement que la taille du pourtour (RB2) de la figure 2B est inférieure à la taille de la base (CB) de la figure 1. Ceci signifie donc une réduction de taille et poids aussi profitable au niveau du couvercle ainsi que pour l'intégration dans une structure de maintien plus étroite tel que c'est souvent le cas en particulier dans des véhicules de tout type (auto, train, avion, soutes,...).

Au même titre de ce dernier avantage, une méthode de fixation de l'ensemble de détection selon l'invention est fortement facilitée en ce qu'une fixation simple, légère et de taille réduite à une structure portante notamment de type plafonnier est effectuée par solidarisation de parties en pourtour (RB2) du couvercle (DCO2) sur au moins un bord correspondant de la structure.

Afin que l'ensemble selon l'invention reste au plus compact, les parois latérales (DCA2) comprennent des bords inférieurs en contact direct avec le couvercle. Ainsi, le dit ensemble présente un nombre minimal de composants formant la cavité allégée.

Enfin, le couvercle possède deux jeux d'ouvertures, chacun des jeux étant disposé directement face à un des capteurs de fumée.

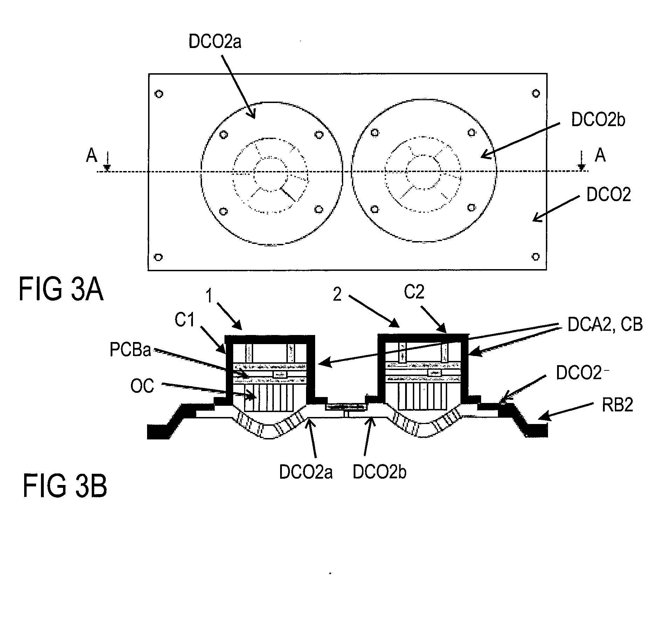

Figures 3A et 3B présentent respectivement une vue de dessous et coupe A-A de face d'un second ensemble de détection selon l'invention comprenant un couvercle (DCO2) tri-bloc.

Principalement, toutes les caractéristiques des figures 2A et 2B décrites précédemment sont communes à celles des figures 3A, 3B.

Aux figures 3A et 3B, il est à noter que la base (CB) c'est-à-dire les deux jeux de parois (DCA2) de chacune des chambres de mesure forment deux compartiments (C1, C2) solidarisables à des éléments internes (DCO2a, DCO2b) au couvercle (DCO2), les dits éléments étant en particulier indépendamment démontables du couvercle (DCO2). Le couvercle a donc ainsi une structure de trois blocs au lieu d'un seul monobloc comme aux figures 2A, 2B. L'avantage lié à cet aspect est de pouvoir démonter plus simplement un seul des détecteurs (par exemple à changer, si un défaut d'un des détecteurs en redondance est signalé/constaté) à la fois sans devoir ouvrir toute la cavité.

## Revendications

1. Ensemble de détection de fumée comprenant :
- deux détecteurs de fumée (1, 2) identiques et juxtaposés horizontalement, chacun composé au moins d'un capteur de fumée disposé sous et couplé à une chambre de mesure (OC) munie de parois (DCA, DCA2),
- les dits détecteurs étant encapsulés dans une cavité rigide ayant une base supérieure (CB) et un couvercle inférieur (CC, DCO, DCO2) disposé face aux capteurs de fumée,
**caractérisée en ce que**
la base (CB) est exclusivement formée par les parois (DCA2) de chacune des deux chambres de mesure.

2. Ensemble selon revendication 1, pour lequel les parois latérales (DCA2) comprennent des bords inférieurs en contact avec le couvercle.

3. Ensemble selon revendication 1 ou 2, pour lequel le couvercle possède deux jeux d'ouvertures, chacun des jeux étant disposé directement face à un des capteurs de fumée.

4. Ensemble selon une des revendications 1 à 3, pour lequel la base (CB, DCA2) comprend deux compartiments (C1, C2) solidarisables à des éléments internes (DCO2a, DCO2b) au couvercle (DCO2), les dits éléments étant en particulier indépendamment démontables du couvercle (DCO2).

5. Ensemble selon une des revendications précédentes, pour lequel la base comporte un matériau résistant aux agressions extérieures spécifiques du lieu (feu, brouillard salins, insecticide..) où l'ensemble est présent, en particulier en fibre de verre ou de carbone.

6. Ensemble selon une des revendications précédentes, pour lequel le couvercle comporte un matériau résistant aux agressions extérieures spécifiques du lieu (feu, brouillard salins, insecticide..) où l'ensemble est présent, particulier en fibre de verre ou de carbone.

7. Ensemble selon une des revendications précédentes, pour lequel la cavité a pour dimension maximale hors tout de 480*480*60mm.

8. Ensemble selon une des revendications précédentes, ayant un poids maximal de 1,5kg.

9. Ensemble selon une des revendications précédentes, pour lequel les détecteurs de fumée forment des capteurs optiques pouvant être combinés avec d'autres types de capteurs tels que de type gaz, thermique, pression.

10. Ensemble selon une des revendications précédentes, pour lequel le couvercle (DCO2) possède un pourtour (RB2) plus étendu qu'un pourtour de la base (DCA2).

11. Méthode de fixation de l'ensemble de détection selon une des revendications précédentes, pour laquelle une fixation à une structure portante notamment de type plafonnier est effectuée par solidarisation de parties en pourtour (RB2) du couvercle (DCO2) sur au moins un bord correspondant de la structure.

## Patentansprüche

1. Rauchmeldeanlage, welche umfasst:
- zwei identische und horizontal nebeneinander angeordnete Rauchmelder (1, 2), die jeweils aus wenigstens einem Rauchsensor bestehen, der unter einer mit Wänden (DCA, DCA2) ausgestatteten Messkammer (OC) angeordnet und mit dieser gekoppelt ist,
- wobei diese Rauchmelder in einen starren Hohlraum eingeschlossen sind, der ein oberes Basisteil (CB) und eine gegenüber den Rauchsensoren angeordnete untere Abdeckung (CC, DCO, DCO2) aufweist,
**dadurch gekennzeichnet, dass**
das Basisteil (CB) ausschließlich von den Wänden (DCA2) jeder der beiden Messkammern gebildet wird.

2. Anlage nach Anspruch 1, wobei die seitlichen Wände (DCA2) untere Ränder umfassen, die sich in Kontakt mit der Abdeckung befinden.

3. Anlage nach Anspruch 1 oder 2, wobei die Abdeckung zwei Sätze von Öffnungen besitzt, wobei jeder der Sätze direkt gegenüber einem der Rauchsensoren angeordnet ist.

4. Anlage nach einem der Ansprüche 1 bis 3, wobei das Basisteil (CB, DCA2) zwei Teilräume (C1, C2) umfasst, die an inneren Elementen (DCO2a, DCO2b) der Abdeckung (DCO2) befestigbar sind, wobei diese Elemente insbesondere unabhängig voneinander von der Abdeckung (DCO2) demontierbar sind.

5. Anlage nach einem der vorhergehenden Ansprüche, wobei das Basisteil ein Material aufweist, das gegenüber den speziellen äußeren aggressiven Einflüssen (Feuer, Salznebel, Insektizid usw.) des Ortes, an dem sich die Anlage befindet, beständig ist, insbesondere ein Material aus Glasfaser oder Kohlenstofffaser.

6. Anlage nach einem der vorhergehenden Ansprüche, wobei die Abdeckung ein Material aufweist, das gegenüber den speziellen äußeren aggressiven Einflüssen (Feuer, Salznebel, Insektizid usw.) des Ortes, an dem sich die Anlage befindet, beständig ist, insbesondere ein Material aus Glasfaser oder Kohlenstofffaser.

7. Anlage nach einem der vorhergehenden Ansprüche, wobei der Hohlraum die maximalen Gesamtabmessungen 480*480*60 mm aufweist.

8. Anlage nach einem der vorhergehenden Ansprüche, welche ein maximales Gewicht von 1,5 kg aufweist.

9. Anlage nach einem der vorhergehenden Ansprüche, wobei die Rauchmelder optische Sensoren bilden, die mit anderen Typen von Sensoren kombiniert werden können, wie Gas-, Temperatur- und Drucksensoren.

10. Anlage nach einem der vorhergehenden Ansprüche, wobei die Abdeckung (DCO2) einen Umfangsrand (RB2) besitzt, der einen weiteren Umfang als ein Umfangsrand des Basisteils (DCA2) aufweist.

11. Verfahren zur Befestigung der Rauchmeldeanlage nach einem der vorhergehenden Ansprüche, wobei eine Befestigung an einer tragenden Struktur, insbesondere vom Typ einer Deckenarmatur, durch starre Befestigung von Abschnitten des Umfangsrandes (RB2) der Abdeckung (DCO2) an wenigstens einem entsprechenden Rand der Struktur erfolgt.

## Claims

1. Smoke detection unit comprising:
- two identical smoke detectors (1, 2) juxtaposed horizontally, each made up of at least one smoke sensor positioned beneath and coupled to a measuring chamber (OC) fitted with walls (DCA, DCA2),
- said detectors being encapsulated in a rigid cavity having an upper base (CB) and a lower cover (CC, DCO, DCO2) positioned opposite the smoke sensors,
**characterised in that**
the base (CB) is exclusively formed by the walls (DCA2) of each of the two measuring chambers.

2. Unit according to claim 1, wherein the side walls (DCA2) comprise lower edges in contact with the cover.

3. Unit according to claim 1 or 2, wherein the cover has two sets of apertures, each of the sets being positioned directly opposite one of the smoke sensors.

4. Unit according to one of claims 1 to 3, wherein the base (CB, DCA2) comprises two compartments (C1, C2) which can be rigidly connected to elements internal (DCO2a, DCO2b) to the cover (DCO2), said elements being in particular independently detachable from the cover (DCO2).

5. Unit according to one of the preceding claims, wherein the base comprises a material resistant to specific external aggressions (fire, saline mist, insecticide, etc.) in the place where the unit is present, in particular glass fibre or carbon.

6. Unit according to one of the preceding claims, wherein the cover comprises a material resistant to specific external aggressions (fire, saline mist, insecticide, etc.) in the place where the unit is present, in particular glass fibre or carbon.

7. Unit according to one of the preceding claims, wherein the cavity has overall maximum dimensions of 480x480x60mm.

8. Unit according to one of the preceding claims, having a maximum weight of 1.5kg.

9. Unit according to one of the preceding claims, wherein the smoke detectors form optical sensors which can be combined with other types of sensors such as of the gas, thermal, pressure type.

10. Unit according to one of the preceding claims, wherein the cover (DCO2) has a more extensive circumference (RB2) than a circumference of the base (DCA2).

11. Method for fixing the detection unit according to one of the preceding claims, wherein fixing to a bearing structure in particular of the ceiling fitting type is performed by rigidly connecting parts on the circumference (RB2) of the cover (DCO2) onto at least one corresponding edge of the structure.
